# EUROPÄISCHE PATENTANMELDUNG

(11) **EP 0 642 803 A2**
(43) Veröffentlichungstag der Anmeldung: **15.03.1995**
(21) Anmeldenummer: 94109923.6
(22) Anmeldetag: 28.06.1994
(51) Int. Cl.: A61M 16/20, A61M 16/04

(54) **Tracheostoma-Verschluss**

(30) Priorität: 11.09.1993 DE 9313765 U
(71) Anmelder: ESKA medical GmbH & Co., D-23556 Lübeck (DE)
(72) Erfinder: Thuaudet, Sylvain, F-14480 Le Fresne Camilly (FR)
(74) Vertreter: Blumbach, Kramer & Partner

(57) **Zusammenfassung**

Es wird ein Tracheostoma-Verschluß beschrieben, der ein zur Ermöglichung des Sprechens durch den Patienten willkürlich betätigbares Ventil, ferner eine in das Stoma des Patienten einführbare Kanüle mit einem Abschlußorgan in der Trachea und ein Ventilgehäuse (1) besitzt, das die Kanüle nach außen abschließt. Um einen guten Halt in der Trachea sicherzustellen, ist das Abschlußorgan als bogenförmiger Fortsatz (4) der Kanüle (2) ausgebildet. Der bogenförmige Fortsatz (4) wird in Richtung zur Lunge in die Trachea eingeführt, so daß die Strömungsverluste beim Atmen klein bleiben und damit ein unbehindertes Atmen möglich ist. Eine Stützplatte (5), die vorzugsweise mit einer angepaßten Öffnung (6) auf die Kanüle (2) aufgeschoben ist, überdeckt auch ein größeres und eingesunkenes Stoma, so daß ein guter Abschluß nach außen auch in schwierigen Fällen erzielt werden kann.

## Beschreibung

Die Erfindung betrifft einen Tracheostoma-Verschluß nach dem Oberbegriff des Anspruchs 1. Einsolcher Verschluß ist beispielsweise bekannt aus der EP-PS 221 973. Der dort offenbart Tracheostoma-Verschluß weist ein vom Patienten willkürlich betätigbares Ventil auf, das in einem Ventilgehäuse untergebracht ist. An das Ventilgehäuse schließt sich ein Kanülenstummel an, der in das Stoma eingeführt wird. Am Ende des Kanülenstummels ist ein Abschlußorgan angebracht, das in die Trachea eingebracht wird und den Verschluß an Ort und Stelle hält. Das Abschlußorgan hat die Form eines an das innere Ende des Kanülenstummels angesetzten, gegen den Kanülenstummel offenen Rohrstücks, das nach oben und unten in die Trachea führt. Das Rohrstück kann dabei auch auf der dem Kanülenstummel gegenüberliegenden Seite vollständig geöffnet sein, also die Form einer Rinne haben. Auf der Außenseite liegt der Tracheostoma-Verschluß mit einem Flanschring an den Rändern des Stoma an, der auf den Kanülenstummel aufgeschoben ist und an das Ventilgehäuse angrenzt.

Der vorstehend beschreibene, bekannte Tracheostoma-Verschluß und ähnlich ausgebildete Verschlüsse haben sich in der Praxis bewährt. Ein möglichst unbehindertes Atmen, also möglichst kleine Strömungsverluste beim Ein- und Ausatmen, wird jedoch weiterhin angestrebt, weil hier oft Probleme subjektiver und objektiver Art bei den Patienten auftreten. Darüber hinaus ergeben sich Schwierigkeiten, wenn das Stoma unregelmäßig begrenzt und eingesunken ist. Das wird insbesondere bei älteren Patienten häufig beobachtet.

Der Erfindung liegt demgemäß die Aufgabe zugrunde, einen Tracheostoma-Verschluß zu schaffen, der ein möglichst unbehindertes Atmen ermöglicht und auch für Patienten mit einem unregelmäßigen und eingesunkenen Stoma geeignet ist.

Die Lösung der Aufgabe ist im Anspruch 1 gekennzeichnet.

Weiterbildungen der Erfindung sind Gegenstand der Unteransprüche.

Der bogenförmige Fortsatz sorgt zum einen als Abschlußorgan für einen guten Halt in der Trachea, ist aber außerdem dem Strömungsverlauf der Atmungsluft angepaßt, so daß die Strömungsverluste klein bleiben und damit ein unbehindertes Atmen möglich ist. Die Stützplatte überdeckt auch ein größeres und eingesunkenes Stoma, so daß ein guter Abschluß nach außen auch in schwierigen Fällen erzielbar ist.

Die Stützplatte ist zweckmäßig am Übergang der Kanüle in das Ventilgehäuse mit einer angepaßten Öffnung aufgeschoben und dort verklebt. Wenn eine zusätzliche Befestigung des Tracheostoma-Verschlusses wegen der anatomischen Verhältnisse beim Patienten angezeigt ist, besitzt die Stützplatte mit Vorteil Öffnungen zur Anbringung von beispielsweise Befestigungsbändern.

Der Außendurchmesser des gekrümmten Fortsatzes sollte an die anatomischen Verhältnisse angepaßt sein. Er kann beispielsweise zwischen 6 und 15 mm betragen, wobei übliche Werte im Bereich zwischen 8 bis 12 mm liegen. Der Gesamtkrümmungswinkel des Fortsatzes kann etwa 90 betragen. Außerdem kann die Wand des Fortsatzes mit Öffnungen in Form von Schlitzen oder Löchern versehen sein, wenn zusätzliche Strömungsquerschnitte erwünscht sind.

Die Stützplatte muß zur Erfüllung ihrer Funktion verhältnismäßig steif sein. Wenn zweckmäßig zur Herstellung Silicon benutzt wird, hat die Stützplatte zweckmäßig eine Dicke von mehreren Millimetern, beispielsweise 2 mm. Die Abdichtung gegen die Haut des Patienten kann dann Schwierigkeiten bereiten, insbesondere bei älteren Patienten mit Falten. Daher sieht eine Weiterbildung der Erfindung vor, daß auf dem bogenförmigen Fortsatz eine mit einer angepaßten Öffnung versehene Folienplatte angeordnet ist, die an der Stützplatte anliegt und über deren Begrenzungen hinausragt. Eine verhältnismäßig dünne Folie schmiegt sich leicht auch Unregelmäßigkeiten der Haut an und sorgt dann für eine gute Abdichtung und ein bequemes Tragen. Zweckmäßig wird dazu die Folienplatte aus weich eingestelltem Silicon hergestellt und hat eine Dicke im Bereich zwischen 0,2 und 0,6 mm. Gegebenenfalls können Belüftungsöffnungen in Form von Perforationen eine übermäßige Schweißbildung verhindern.

Nicht nur die Folienplatte, sondern alle Teile des Tracheostoma-Verschlusses werden zweckmäßig aus einem für medizinische Zwecke zugelassenen Silicon hergestellt.

Nachfolgend wird ein Ausführungsbeispiel der Erfindung anhand der Zeichnung beschrieben. Es zeigen:
Fig. 1 die schematische Seitenansicht eines Tracheostoma-Ventils nach der Erfindung;
Fig. 2 die Vorderansicht der Stützplatte für das Ventil nach Fig. 1;
Fig. 3 die Ansicht einer Folienplatte für das Ventil nach Fig. 1.

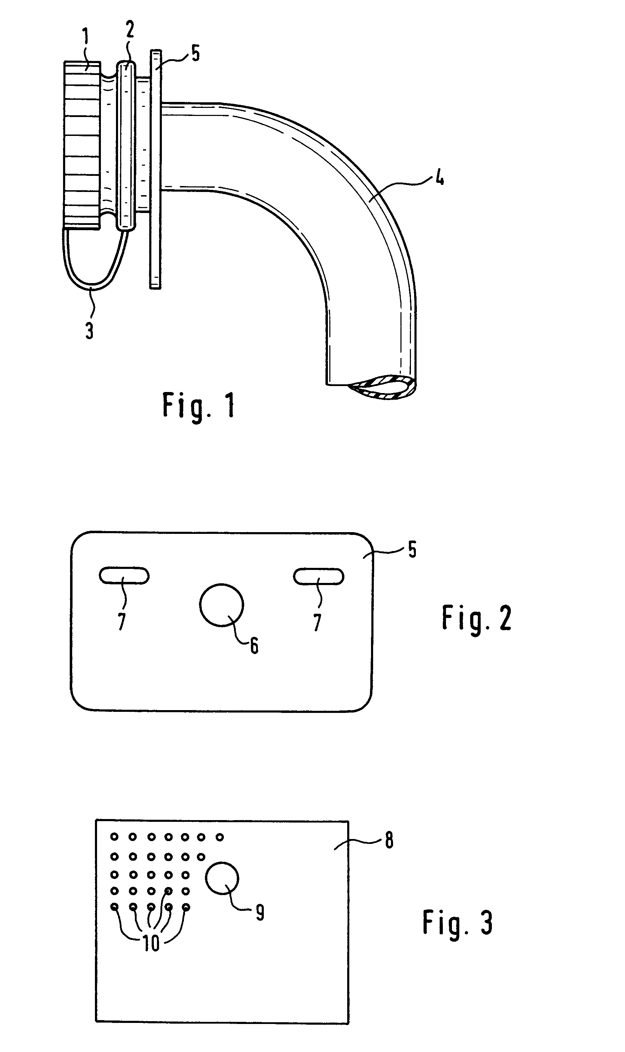

Der Tracheostoma-Verschluß gemäß Fig. 1 besitzt ein abnehmbares Ventilgehäuse 1, das auf einen Kanülenstummel 2 aufgesetzt ist und mittels eines Bandes 3 unverlierbar gehalten wird. Sowohl das Ventillgehäuse als auch die Einzelteile des Ventils selbst können entsprechend dem Ventil nach der vorgenannten EP-PS 221 973 ausgeführt sein und werden daher nicht im einzelnen erläutert.

Es können in gleicher Weise aber auch andere Ventile benutzt werden. Vom Ventilgehäuse 1 und dem Kanülenstummel 2 führt ein bogenförmiger

Fortsatz 4 bei eingesetztem Verschluß in die Trachea des Patienten, und zwar in Richtung zur Lunge. Den Abschluß nach außen bildet eine Stützplatte 5, die auf den Fortsatz 4 aufgeschoben ist. Dazu weist, wie Fig. 2 zeigt, die Stützplatte 5 eine zentrale Öffnung 6 auf, deren Durchmesser an den Durchmesser des Fortsatzes 4 angepaßt ist. Zur Vereinfachung der Darstellung ist, wie die Abmessungen der Öffnung 6 und des Fortsatzes 4 zeigen, die Stützplatte 5 in Fig. 2 gegenüber Fig. 1 verkleinert dargestellt. Langlöcher 7 ermöglichen die sichere Festlegung der Stützplatte 5 und damit des Gesamtverschlusses am Hals des Patienten mit Hilfe von Bändern, die durch die Langlöcher 7 geführt sind.

In Fig. 3 ist wiederum gegenüber der Darstellung in Fig. 2 verkleinert eine Folienplatte 8 gezeigt, die mit einer angepaßten aufgeschoben wird und hinter der Stützplatte 5 an dieser anliegt. Die Folienplatte, die wie alle anderen Bestandteile des Verschlusses aus Silicon besteht, hat eine Dicke von beispielsweise 0,3 mm und kann sich daher gut auch an Unregelmäßigkeiten der Haut anschmiegen. Die Folienplatte 8 hat beispielsweise Abmessungen von 90 x 110 mm, ist also wesentlich größer als die Stützplatte 5, deren Abmessungen beispielsweise 40 x 60 mm betragen. Die Abmessungen der Stützplatte können je nach den anatomischen Verhältnissen eine Breite zwischen 40 und 80 mm und eine Höhe von 30 bis 50 mm haben. Auch die Folienplatte kann größere oder kleinere Werte annehmen. Die in der linken oberen Ecke der Folienplatte 8 angedeuteten Löcher oder Perforationen 10 sorgen für eine Belüftung der Haut des Patienten und verhindern daher ein übermäßiges Schwitzen.

## Patentansprüche

1. Tracheostoma-Verschluß mit einem zur Ermöglichung des Sprechens von Patienten willkürlich betätigbaren Ventil, einer in das Stoma einführbaren Kanüle, die ein in die Trachea einbringbares Abschlußorgan besitzt, und einem Ventilgehäuse, das die Kanüle nach außen abschließt,
dadurch gekennzeichnet,
daß das Abschlußorgan als ein bogenförmiger Fortsatz (4) der Kanüle (2) ausgebildet ist, der in Richtung der Lunge in die Trachea einführbar ist, und
daß die Kanüle in Richtung zum Ventilgehäuse (1) eine Stützplatte (5) aufweist, die die Umgebung des Stoma außen abdeckt.

2. Tracheostoma-Verschluß nach Anspruch 1, dadurch gekennzeichnet, daß die Stützplatte (5) am Übergang der Kanüle (2) in das Ventilgehäuse (1) mit einer angepaßten Öffnung (6) auf die Kanüle aufgeschoben und dort verklebt ist.

3. Tracheostoma-Verschluß nach Anspruch 1 oder 2,
dadurch gekennzeichnet, daß die Stützplatte (5) generell rechteckig ausgebildet ist, eine Breite im Bereich von 40 bis 80 mm, eine Höhe im Bereich von 30 bis 50 mm und eine Dicke zwischen 1 und 3 mm besitzt.

4. Tracheostoma-Verschluß nach einem der Ansprüche 1 bis 3, dadurch gekennzeichnet, daß die Stützplatte (5) Öffnungen (7) zur Anbringung von Befestigungsbändern besitzt.

5. Tracheostoma-Verschluß nach einem der Ansprüche 1 bis 4,
dadurch gekennzeichnet, daß der gekrümmte Fortsatz (4) einen Außendurchmesser im Bereich zwischen 6 und 15 mm, vorzugsweise 8 bis 12 mm besitzt.

6. Tracheostoma-Verschluß nach einem der Ansprüche 1 bis 5,
dadurch gekennzeichnet, daß der Fortsatz (4) einen Krümmungsradius von etwa 90 besitzt.

7. Tracheostoma-Verschluß nach einem der Ansprüche 1 bis 6,
dadurch gekennzeichnet, daß die Wand des Fortsatzes (4) mit Öffnungen in Form von Schlitzen oder Löchern versehen ist.

8. Tracheostoma-Verschluß nach einem der Ansprüche 1 bis 7,
dadurch gekennzeichnet, daß auf den bogenförmigen Fortsatz (4) eine mit einer angepaßten Öffnung versehene Folienplatte (8) angeordnet ist, die an der Stützplatte (5) anliegt und über deren Begrenzungen hinausragt.

9. Tracheostoma-Verschluß nach Anspruch 8, dadurch gekennzeichnet, daß die Folienplatte (8) aus weich eingestelltem Silicon besteht und eine Dicke zwischen 0,2 und 0,6 mm, vorzugsweise 0,3 bis 0,4 mm besitzt.

10. Tracheostoma-Verschluß nach Anspruch 8 oder 9,
dadurch gekennzeichnet, daß die Folienplatte (8) mit Belüftungsöffnungen (10) versehen ist.
